# EUROPEAN PATENT SPECIFICATION

(11) **EP 3 013 423 B1**
(45) Date of publication and mention of the grant of the patent: **22.08.2018**
(21) Application number: 14734550.8
(22) Date of filing: 09.05.2014
(51) Int. Cl.: A61P 17/00, A61P 17/02, A61K 36/73, A61K 36/185, A61K 8/92, A61K 36/736, A61K 36/738, A61K 36/28, A61K 36/63, A61K 47/44, A61K 35/36, A61Q 19/00

(54) **COMPOSITION COMPRISING OLEUM COCTUS URTICAE**
ZUSAMMENSETZUNG MIT OLEUM COCTUS URTICAE
COMPOSITION COMPRENANT OLEUM COCTUS URTICAE

(30) Priority: 25.06.2013 IT MI20131053
(43) Date of publication of application: 04.05.2016
(73) Proprietor: Pandela s.r.l., I-20145 Milano (IT)
(72) Inventor: Salviati, Maria Oliva, 20121 Milano (IT)
(74) Representative: Rigamonti, Dorotea
(86) International application number: PCT/IB2014/061337
(87) International publication number: WO 2014/207584

(56) References cited:
- EP-A1- 2 574 343
- WO-A1-2006/015675
- WO-A1-2011/104200
- DE-U1-202006 019 183
- US-A1- 2005 249 830
- DATABASE WPI Week 200025 Thomson Scientific, London, GB; AN 2000-291326 XP002717964, & RU 2 127 584 C1 (KONOVALOV V N) 20 March 1999 (1999-03-20)
- DATABASE WPI Week 200116 Thomson Scientific, London, GB; AN 2001-157199 XP002717965, & RU 2 153 321 C1 (KORA CO STOCK CO) 27 July 2000 (2000-07-27)
- Floleads.com: "Herbal Oils", , 1 January 1995 (1995-01-01), XP002717986, Retrieved from the Internet: URL:http://floraleads.com/EVENING.HTM#nett le [retrieved on 2013-12-16]
- SHYAMALA B N ET AL: "Leafy vegetable extracts-antioxidant activity and effect on storage stability of heated oils", INNOVATIVE FOOD SCIENCE AND EMERGING TECHNOLOGIES, ELSEVIER, AMSTERDAM, NL, vol. 6, no. 2, 1 June 2005 (2005-06-01), pages 239-245, XP027837727, ISSN: 1466-8564 [retrieved on 2005-06-01]
- DATABASE WPI Week 199410 Thomson Scientific, London, GB; AN 1994-079392 XP002728937, & JP H06 31107 A (JUKI CORP) 8 February 1994 (1994-02-08)
- WANG L ET AL: "Recent advances in extraction of nutraceuticals from plants", TRENDS IN FOOD SCIENCE AND TECHNOLOGY, ELSEVIER SCIENCE PUBLISHERS, GB, vol. 17, no. 6, 1 June 2006 (2006-06-01), pages 300-312, XP027935129, ISSN: 0924-2244 [retrieved on 2006-06-01]
- Floraleads: "Herbal Oils Questions and Answers", , 1 January 1995 (1995-01-01), XP002717987, Retrieved from the Internet: URL:http://floraleads.com/ADVICE.HTM [retrieved on 2013-12-16]

## Description

The present invention relates to a composition comprising one or more oils and *Oleum coctus Urticae.*

A further object of the present invention is a method of preparing said composition and the use thereof in the treatment and/or prevention of skin diseases and of physiological degenerative processes of the skin in mammals.

### Background of the art

There are hundreds of skin diseases, and some of them may present with apparently similar or overlapping clinical manifestations.

The group encompasses, *inter alia,* skin and subcutaneous tissue infections, bullous diseases, dermatitis and eczema, papulosquamous disorders, urticaria and erythema, photodermatoses and skin changes by ionizing radiations, pigmentation disorders, decubitus ulcer, fibromatosis, lupus erythematosus, skin disorders during vasculitis and other angiopathies, skin malignancies, benign skin tumors.

In addition to manifestations of a pathological type, skin incurs degenerative processes of a physiological type, related to age progression.

The need is strongly felt, of compositions capable of recovering the skin degenerations, whether they are of pathological or a physiological type.

Unguents, composed of a low water content base, are indicated in the treatment of dry, squamous dermatoses. In fact, the fat base, by virtue of its occlusive properties, exerts an advantageous wetting effect, allowing a deeper action of the medicament; the unguents are particularly suitable to skin conditions that may benefit from a prolonged massage and are characterized by a slow absorption. Oil and wax-base unguents are known, where from 5 to 7 parts of oil or oil mixture are combined to one part of bee wax. Unguents are also known, where from 7 to 10 parts of oil or oil mixture are combined with one part of vegetal wax.

Nettle, which is a plant of the genus *Urtica,* in addition to the known allergic skin reaction, is known in pharmacognosis for its antiphlogistic, diuretic, prostatic properties.

The nettle fruits contain oils, mucilages, and proteins; the aerial parts contain flavonoids, vitamins, chlorophyll, mineral salts, and secretin; its stinging hairs contain histamine, acetylcholine, and formic acid, which is responsible for the stinging effect. Finally, nettle root contains phenols, tannin, sterols, lecithin, and polysaccharides.

Fats contained in the seeds are provided with restorative properties and are used in cases of asthenia; recent studies showed that the nettle root has beneficial effects in case of prostatic hypertrophy, resulting in a reduction in the gland volume. Also a diuretic and anti-inflammatory action is associated with the same root. The nettle leaves, due to the presence of mineral salts and chlorophyll, would have an anti-anaemic action. The nettle juice invigorates and stimulates the scalp. It is used against seborrhea and hair loss.

Hair care nettle-based shampoos are currently available, in addition to formulations comprising nettle for internal use.

RU2127584 (Konovalov V N, 20/03/1999) describes nettle alcoholic extracts for use for the treatment of skin disorders.

RU2153321 (Kora Co Stock Co, 27/07/2000) describes nettle oil extracts for use for the treatment of skin disorders.

WO 2006/015675 describes skin cosmetics.

In Floreads.com: "Herbal Oils" (01/01/1995) nettle for use for the treatment of skin disorders is disclosed.

### Description of the invention

The present invention discloses a composition comprising one or more oils and *Oleum coctus Urticae,* according to claim 1.

Said composition finds use in the prevention and/or in treatment of skin diseases and physiological degenerative processes of the skin in mammals.

### Detailed description of the invention

The present invention discloses a composition comprising one or more oils and *Oleum coctus Urticae,* according to claim 1.

In a preferred embodiment, said composition comprises, in addition, one or more waxes.

By nettle is meant herein the plant of the genus *Urtica,* in one of the *dioica, urens,* or *pilulifera* species. To the purposes of the present invention, the raw plant, aerial part, and/or roots are used.

By the term *Oleum coctus Urticae* is meant herein the nettle extract obtained by immersing the raw plant, aerial part, and/or roots, in hot oil. The plant is macerated during a few minutes; subsequently, one proceeds with filtration to remove the plant residues.

Said composition comprises, per each liter of oil or oil mixture, *Oleum coctus Urticae* obtained from 1 - 500 g of raw plant, aerial part, and/or roots of nettle, or 10 to 250 g, or 15 to 185 g, or 20 to 100 g, or 25 to 60 g, or 30 to 50 g, preferably from 35 g of raw plant, aerial part, and/or roots of nettle.

In a preferred embodiment, where said composition comprises also one or more waxes, said composition comprises, per one part of wax or wax mixture, 7 to 25 parts by weight of oil, or oil mixture, and *Oleum coctus Urticae* obtained from 0.10 - 3 parts by weight of raw plant, aerial part, and/or roots of nettle. Preferably, said composition comprises from 8 to 20 parts of oil or oil mixture and *Oleum coctus Urticae* from 0.30 - 2 parts by weight of nettle, or from 10 to 15 parts of oil or oil mixture and *Oleum coctus Urticae* from 0,40 - 1 part of nettle. In a preferred embodiment, said composition comprises about 11.4 parts of oil or oil mixture and *Oleum coctus Urticae* from about 0.44 parts by weight of nettle per one part of wax.

Said one or more oils are preferably vegetal oils, selected, by way of non-limiting example, from argan oil, almond oil, rose hip oil, shea oil, chamomile oil, avocado oil, lemon oil, wheat germ oil, carrot oil, calendola oil, oil of Hypericum, ivy, coconut, macassar, hazelnut, pomegranate, lavender cotton, Helichrysum, baobab, apricot, sesame, sunflower, walnut, corn, olive. In a preferred embodiment, said oil is olive oil.

Said one or more waxes are synthetic or natural waxes. By way of example, said synthetic wax may be isopropyl myristate. Preferably, they are one or more natural waxes, selected from waxes of vegetal origin, such as, by way of example, carnauba wax and Jojoba oil, or of animal origin, such as, by way of example, bee wax, spermaceti, uropigyal ester, and lanolin. In a preferred embodiment, said wax is bee wax.

The composition of the present invention may be comprised in a dermatological/dermal-cosmetic preparation that may comprise, in addition, suitable pharmaceutically acceptable excipients, such as antioxidant substances, for example Vitamin E, gelling substances, emulsifiers, diluents, or other additives known in the pharmaceutics art.

Said composition may further comprise one or more active ingredients of natural or synthetic origin.

For description purpose only, said composition will be able to comprise a cell suspension representative of one or more epidermal cell types. By way of example, a cell suspension will be able to be used, comprising undifferentiated basal cells and/or keratinocytes and/or melanocytes and/or fibroblasts and/or Langerhans cells. Said cells will be heterologous cells, or preferably, they will be autologous cells.

The composition of the present invention is prepared according to the following procedure.

The oil or oil mixture is heated at a temperature ranging between 140 and 180° C, preferably about 160° C. The raw plant, aerial part, and/or roots of nettle are then added to the hot oil, and it is left macerating from at least 1 minute up to 3 hours, or up to 1 hour, or up to 40 minutes, or up to 20 minutes, or up to 10 minutes. Preferably, maceration is carried out during about 3 minutes. Then, one proceeds with a filtration so as to remove the plant residues, and the filtrate is the composition of the present invention.

Where said composition also comprises one or more waxes, the wax or wax mixture is added to the preparation at the end of said maceration step. The composition is suitably mixed to melt the wax or wax mixture; then, a filtration is carried out. The filtrate is the composition at issue.

It is a further aspect of the present invention a method of preparing extracts from raw plants, comprising the following steps:
an oil or oil mixture is heated at a temperature ranging between 140 and 180° C, preferably about 160° C;
a raw plant, aerial part, and/or roots is added to said hot oil or oil mixture, which is left macerating from at least 1 minute up to 3 hours, or up to 1 hour, or up to 40 minutes, or up to 20 minutes, or up to 10 minutes; preferably, maceration is carried out during about 3 minutes;
the mixture is filtered, and the filtrate is the extract.

Said extract has unique characteristics in terms of active ingredients extracted compared to extracts obtained by method that are known in the state of the art; furthermore, said extract has the characteristic of a prolonged stability, even in non-optimal environments conditions.

The composition of the present invention finds application in treating or prevention of cutaneous degenerative phenomena, of a pathological or physiological type, in a mammal, preferably humans, dog, cat, horses, pigs, and cattle.

Said composition is used, by way of non-limiting example only, in the treatment of skin and subcutaneous tissue infections, septic injuries, blast injuries, insect stings, bullous diseases, dermatitis and eczema, papulosquamous disorders, urticaria and erythema, photodermatoses and skin changes by ionizing radiations, pigmentation disorders, decubitus ulcer, fibromatosis, lupus erythematosus, skin disorders during vasculitis and other angiopathies, skin malignancies, benign skin tumors, skin disorders of psoriasis. Said composition finds also application in skin complications due to treatment with corticoids, cytostatic agents, retinoic acid and derivatives thereof, or with other natural substances and/or synthetic substances. In particular, said composition is useful in the treatment of I, II, III, and IV degree burns, atopic dermatitis, skin disorders of psoriatic arthritis, in the treatment of phlyctenae, tyloma, skin ulcers, decubitus ulcers. Said composition finds also use in vascular diseases conditions, such as trophic ulcer, diabetes ulcer, skin vascular necrosis, anal rhagades, hemorrhoids, ulcers, or phlogosis of vaginal mucosae.

Said composition finds also use as a co-adjuvant in therapy. Preferably, said therapy is selected from therapies providing for the use of laser or of ultraviolet radiations or photochemotherapies, surgeries, chemotherapy, radiotherapy, treatments with corticoids and/or other natural and/or synthetic substances for topic use. When used as a co-adjuvant, the application may be carried out concomitantly with or delayed with respect to the further treatment, according to the subject's needs.

The composition of the present invention finds further use in the prevention and in treatment of skin aging.

The frequency of application of said composition or the dermatological/dermal-cosmetic preparation comprising it varies according to the subject's needs. Said frequency may range between once per month and 10 times a day, preferably between once a week and 4 times a day, more preferably between 3 times a week and 3 times a day, still more preferably 1 or 2 times a day.

As best illustrated in the following examples, the effects obtained by the use of the composition of the present invention are surprising.

Said composition, used for the treatment of first degree burns, results in the disappearance of pain within minutes. When it is used for the treatment of second and third degree burns, it results in a complete remission of pain within 12/24 hours. In addition, subsequent the inflammation and infection sensibly decrease within 48 hours from the treatment until reaching a full healing.

The use of said composition in trophic ulcers, also known by the name of varicose ulcers, results, within 24 hours, in a recovery of about 20% and, within 15 days from the beginning of the treatment, recovery is of about 70%.

Surprisingly, when it is used in the treatment of advanced-stage decubitus ulcers, already purulent, said composition results in a recovery of about 50% after 30 days of treatment and the recovery is almost complete after 2 months of treatment.

### Description of the Figures

Fig.1: painful trophic ulcer of V metatarsal before treatment.
Fig. 2: painful trophic ulcer of the V metatarsal after 24 hours and after 15 days of treatment.
Fig. 3: necrotic vascular trophic ulcer before treatment.
Fig. 4: necrotic vascular trophic ulcer after 10, 28 days and 3 months of treatment.
Fig. 5: decubitus ulcer before treatment.
Fig. 6: decubitus ulcer after 30, 40, 50, and 60 days of treatment.
Fig. 7: ulcer due to plaster cast device.
Fig. 8: ulcer due to plaster cast device after 30 days of treatment.

### EXAMPLES

### Example 1: preparation of a composition comprising olive oil, bee wax, and Oleum coctus Urticae.

1 liter olive oil is heated to a temperature of about 160° C. When the oil has reached the temperature indicated, 35 g of nettle, the whole raw plant, is added, and left during about 3 minutes. At the end, 80 g bee wax is added to the preparation. The preparation is mixed so as to dissolve the bee wax; subsequently, a filtration is carried out, thus obtaining a filtrate that, once cooled, is the ready-to-use composition.

### Example 2: use of a composition comprising Oleum coctus Urticae in the treatment of a vascular trophic ulcer of the V metatarsal bone.

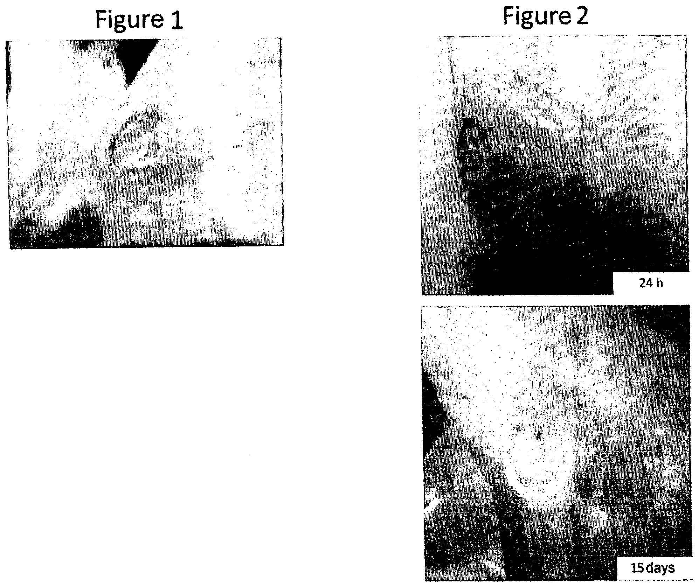

A composition prepared according to the example 1 is applied onto the area affected from a painful vascular trophic ulcer of the V metatarsal, with appearance as in Fig. 1. In the following days, one proceeds with the application of the same composition twice a day. 24 hours after the first treatment, pain is absent. On day 7 of treatment, the ulcer appears as in Fig. 2. On day 15 of treatment, the ulcer is clinically healed, as shown by the image set forth in the same Fig. 2.

### Example 3: use of a composition comprising Oleum coctus Urticae in the treatment of a necrotic vascular trophic ulcer.

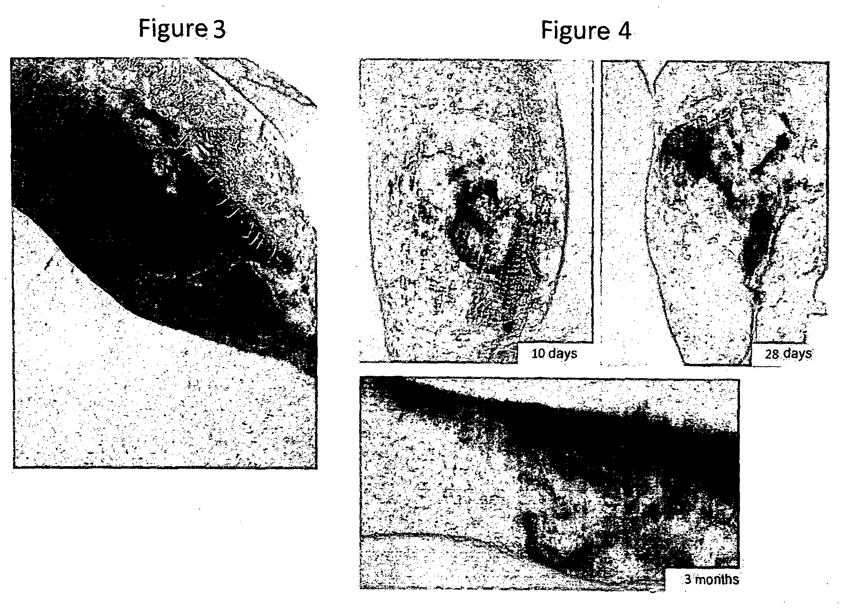

A diabetic subject with vascular disease has an infected necrotic vascular trophic ulcer on a kneel prosthesis. Said ulcer is, before the treatment, as illustrated in Fig. 3. The composition prepared as described in the example 1 is applied on the part at issue. During the following days, one proceeds with the application of the same composition twice a day. On day 10, a recovery can be noticed, as shown in Fig. 4. Said recovery is consistent on day 28 of treatment, Fig. 4. At 3 months, the recovery is clear, Fig. 4.

### Example 4: use of a composition comprising Oleum coctus Urticae in the treatment of a decubitus ulcer.

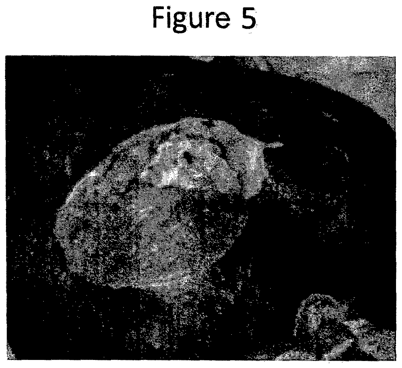

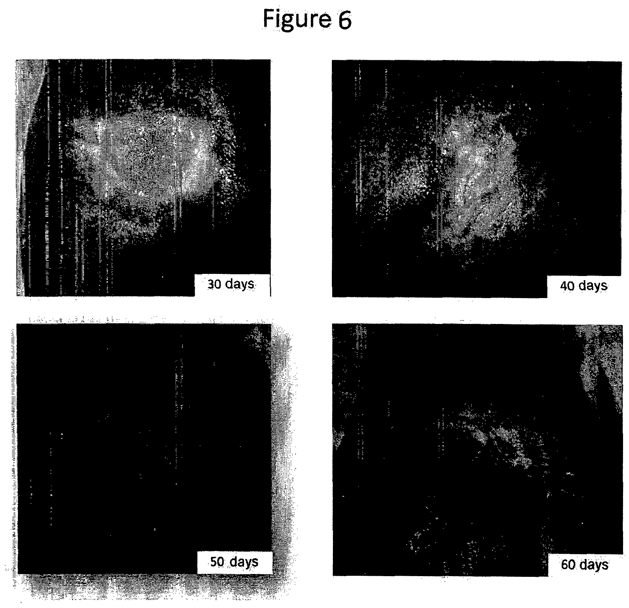

A 12-years old child affected from spastic palsy, has decubitus ulcers with substantial dimensions, as illustrated in Fig. 5. The composition prepared as described in the example 1 is applied on the part at issue, and the treatment is repeated during the next 60 days, with applications twice a day. Fig. 6 sets forth the recovery noticed at 30, 40, 50 and 60 days. Surprisingly, recovery is complete within day 60.

### Example 5: use of a composition comprising Oleum coctus Urticae in the treatment of an ulcer due to a plaster cast device

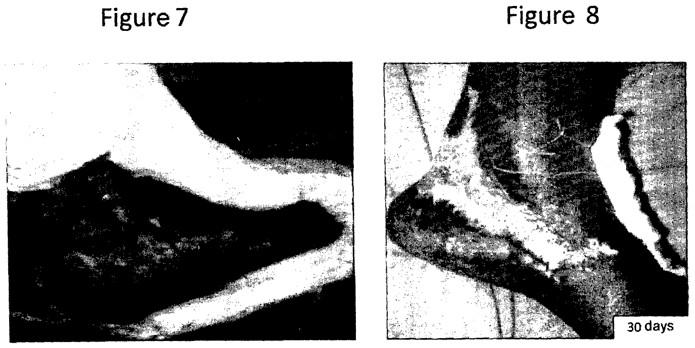

An ulcer due to a plaster cast device as illustrated in Fig. 7 is treated with the composition prepared as described in the example 1. The treatment is repeated during the next 60 days, with applications twice a day. Fig. 8 sets forth the recovery noticed at 30 and 60 days. Surprisingly, recovery is complete within day 60.

## Claims

1. A composition comprising one or more oils and *Oleum coctus Urticae.*

2. The composition according to claim 1 comprising, per each liter of oil or oil mixture, *Oleum coctus Urticae* obtained from 1 - 500 g of raw plant, aerial part, and/or roots of nettle, or 10 to 250 g, or 15 to 185 g, or 20 to 100 g, or 25 to 60 g, or 30 to 50 g, preferably, per each liter of oil or oil mixture, *Oleum coctus Urticae* obtained from 35 g of raw plant, aerial part, and/or roots of nettle.

3. The composition according to one of the claims 1 or 2, also comprising one or more waxes.

4. The composition according to claim 3, comprising, per one part of wax or wax mixture, 7 to 25 parts by weight of oil or oil mixture and *Oleum coctus Urticae* obtained from 0.10 - 3 parts by weight of raw plant, aerial part, and/or roots of nettle, preferably from 8 to 20 parts of oil or oil mixture and *Oleum coctus Urticae* from 0.30 - 2 parts by weight of nettle, still more preferably, from 10 to 15 parts of oil or oil mixture and *Oleum coctus Urticae* from 0,40 - 1 part of nettle.

5. The composition according to one of the claims 3 or 4, comprising, per one part of wax or wax mixture, about 11.4 parts of oil or oil mixture and *Oleum coctus Urticae* obtained from about 0.44 parts by weight of raw plant, aerial part, and/or roots of nettle.

6. The composition according to one of the claims 1 to 5, where said one or more oils are vegetal oils selected from the group comprising argan oil, almond oil, rose hip oil, shea oil, chamomile oil, avocado oil, lemon oil, wheat germ oil, carrot oil, calendola oil, oil of Hypericum, ivy, coconut, macassar, hazelnut, pomegranate, lavender cotton, Helichrysum, baobab, apricot, sesame, sunflower, walnut, corn, olive.

7. The composition according to one of the claims 1 to 6, where said oil is olive oil.

8. The composition according to one of the claims 3 to 7, where said one or more waxes are synthetic or natural waxes, of vegetal origin or animal origin.

9. The composition according to one of the claims 3 to 7, where said wax is bee wax.

10. The composition according to one of the claims 1 to 9, also comprising one or more further active ingredients of natural or synthetic origin.

11. A method of preparing extracts from raw plants, wherein the plant is nettle, where said method comprises:
- providing one or more oils and heating thereof at a temperature ranging from 140 to 180 °C, preferably about 160 °C;
- adding the raw plant, aerial part, and/or roots of nettle;
- macerating from at least 1 minute up to 3 hours, or up to 1 hour, or up to 40 minutes, or up to 20 minutes, or up to 10 minutes, preferably during about 3 minutes;
- filtrating, where the filtrate is said composition.

12. The method according to claim 11, wherein said plant is nettle and said extract is a composition comprising one or more oils and *Oleum coctus Urticae.*

13. The method according to claims 11 or 12, also comprising, after said maceration step and before carrying out said filtration:
- adding one or more waxes;
- mixing the composition.

14. The method according to one of the claims from 11 to 13, where said one or more oils are vegetal oils selected from the group comprising argan oil, almond oil, rose hip oil, shea oil, chamomile oil, avocado oil, lemon oil, wheat germ oil, carrot oil, calendola oil, Hypericum oil, oil of ivy, of coconut, macassar, hazelnut, pomegranate, lavender cotton, Helichrysum, baobab, apricot, sesame, sunflower, walnut, corn, olive.

15. The method according to claim 13, where said one or more waxes are synthetic or natural waxes, of vegetal origin or animal origin.

16. The composition according to one of the claims 1 to 10, for use in treating or prevention of cutaneous degenerative phenomena, of a pathological type, in a mammal.

17. The composition for use according to claim 16, where said phenomena are skin and subcutaneous tissue infections, septic injuries, blast injuries, insect stings, bullous diseases, dermatitis and eczema, papulosquamous disorders, urticaria and erythema, photodermatoses and skin changes by ionizing radiations, pigmentation disorders, decubitus ulcer, fibromatosis, lupus erythematosus, skin disorders during vasculitis and other angiopathies, skin malignancies, benign skin tumors, skin disorders of psoriasis; skin complications due to treatment with corticoids, cytostatic agents, retinoic acid and derivatives thereof, or with other natural substances and/or synthetic substances; I, II, III, and IV degree burns, atopic dermatitis, skin disorders of psoriatic arthritis, phlyctenae, tyloma, trophic ulcer, diabetes ulcer, skin vascular necrosis, anal rhagades, hemorrhoids, ulcers and/or phlogosis of vaginal mucosae.

18. The composition according to one of the claims 1 to 10, for use as a co-adjuvant in therapy to prevent skin damages, where said therapy is selected from the group comprising therapies providing for the use of laser or of ultraviolet radiations or photochemotherapies, surgeries, chemotherapy, radiotherapy, treatments with corticoids and/or other natural and/or synthetic substances for topic use.

19. A dermatological/dermal-cosmetic preparation comprising the composition according to one of the claims 1 to 10 and pharmaceutically acceptable excipients.

20. A composition comprising one or more oils and *Oleum coctus Urticae* prepared according to the method of the claims 11 to 15.

21. Use of a composition according to claims 1-10 as a cosmetic composition for the treatment of skin ageing.

## Patentansprüche

1. Zusammensetzung, umfassend ein oder mehrere Öle und *Oleum coctus Urticae.*

2. Zusammensetzung nach Anspruch 1 umfassend, pro Liter Öl oder Ölmischung, *Oleum coctus Urticae,* erhalten aus 1 - 500 g roher Pflanze, oberirdischem Teil und/oder Wurzeln der Nessel, oder 10 bis 250 g, oder 15 bis 185 g, oder 20 bis 100 g, oder 25 bis 60 g, oder 30 bis 50 g, vorzugsweise, pro Liter Öl oder Ölmischung, *Oleum coctus Urticae,* erhalten aus 35 g roher Pflanze, oberirdischem Teil und/oder Wurzeln der Nessel.

3. Zusammensetzung nach einem der Ansprüche 1 oder 2, auch umfassend ein oder mehrere Wachse.

4. Zusammensetzung nach Anspruch 3, umfassend, pro ein Teil Wachs oder Wachsgemisch, 7 bis 25 Gewichtsteile Öl oder Ölmischung und *Oleum coctus Urticae,* erhalten aus 0,10 - 3 Gewichtsteilen roher Pflanze, oberirdischem Teil und/oder Wurzeln der Nessel, vorzugsweise von 8 bis 20 Teilen Öl oder Ölmischung und *Oleum coctus Urticae* aus 0,30 - 2 Gewichtsteilen Nessel, noch stärker bevorzugt von 10 bis 15 Teilen Öl oder Ölmischung und *Oleum coctus Urticae* aus 0,40 - 1 Teil Nessel.

5. Zusammensetzung nach einem der Ansprüche 3 oder 4, umfassend, pro ein Teil Wachs oder Wachsgemisch, etwa 11,4 Teile Öl oder Ölgemisch und *Oleum coctus Urticae,* erhalten aus etwa 0,44 Gewichtsteilen roher Pflanze, oberirdischem Teil und/oder Wurzeln der Nessel.

6. Zusammensetzung nach einem der Ansprüche 1 bis 5, wobei das eine oder die mehreren Öle pflanzliche Öle sind, ausgewählt aus der Gruppe umfassend Arganöl, Mandelöl, Hagebuttenöl, Sheaöl, Kamillenöl, Avocadoöl, Zitronenöl, Weizenkeimöl, Karottenöl, Calendulaöl, Öl von Johanniskraut, Efeu, Kokosnuss, Makassar, Haselnuss, Granatapfel, Lavendel, Baumwolle, Strohblume, Affenbrotbaum, Aprikose, Sesam, Sonnenblume, Walnuss, Mais, Olive.

7. Zusammensetzung nach einem der Ansprüche 1 bis 6, wobei das Öl Olivenöl ist.

8. Zusammensetzung nach einem der Ansprüche 3 bis 7, wobei das eine oder die mehreren Wachse synthetische oder natürliche Wachse pflanzlichen Ursprungs oder tierischen Ursprungs sind.

9. Zusammensetzung nach einem der Ansprüche 3 bis 7, wobei das Wachs Bienenwachs ist.

10. Zusammensetzung nach einem der Ansprüche 1 bis 9, auch umfassend einen oder mehrere weitere Wirkstoffe natürlichen oder synthetischen Ursprungs.

11. Verfahren zur Herstellung von Extrakten aus Rohpflanzen, wobei die Pflanze Nessel ist,
wobei das Verfahren umfasst:
- Bereitstellen eines oder mehrerer Öle und Erwärmen auf eine Temperatur im Bereich von 140 bis 180°C, vorzugsweise etwa 160°C;
- Zugabe der rohen Pflanze, des oberirdischen Teils und/oder der Wurzeln der Nessel;
- Mazerieren von mindestens 1 Minute bis zu 3 Stunden oder bis zu 1 Stunde oder bis zu 40 Minuten oder bis zu 20 Minuten oder bis zu 10 Minuten, vorzugsweise während etwa 3 Minuten;
- Filtrieren, wobei das Filtrat die Zusammensetzung ist.

12. Verfahren nach Anspruch 11, wobei die Pflanze Nessel ist und der Extrakt eine Zusammensetzung ist, die ein oder mehrere Öle und *Oleum coctus Urticae* umfasst.

13. Verfahren nach Anspruch 11 oder 12, auch umfassend nach dem Mazerationsschritt und vor Durchführung der Filtration:
- Zugabe eines oder mehrerer Wachse;
- Mischen der Zusammensetzung.

14. Verfahren nach einem der Ansprüche 11 bis 13, wobei das eine oder die mehreren Öle pflanzliche Öle sind, ausgewählt aus der Gruppe umfassend Arganöl, Mandelöl, Hagebuttenöl, Sheaöl, Kamillenöl, Avocadoöl, Zitronenöl, Weizenkeimöl, Karottenöl, Calendulaöl, Johanniskrautöl, Öl von Efeu, Kokosnuss, Makassar, Haselnuss, Granatapfel, Lavendel, Baumwolle, Strohblume, Affenbrotbaum, Aprikose, Sesam, Sonnenblume, Walnuss, Mais, Olive.

15. Verfahren nach Anspruch 13, wobei das eine oder die mehreren Wachse synthetische oder natürliche Wachse pflanzlichen Ursprungs oder tierischen Ursprungs sind.

16. Zusammensetzung nach einem der Ansprüche 1 bis 10 zur Verwendung bei der Behandlung oder Vorbeugung von kutanen degenerativen Phänomenen pathologischen Typs in einem Säugetier.

17. Zusammensetzung zur Verwendung nach Anspruch 16, wobei die Phänomene Infektionen der Haut und des subkutanen Gewebes, septische Verletzungen, Explosionsverletzungen, Insektenstiche, bullöse Erkrankungen, Dermatitis und Ekzeme, papulosquamöse Störungen, Urtikaria und Erythem, Photodermatosen und Hautveränderungen durch ionisierende Strahlungen, Pigmentstörungen, Dekubitalgeschwür, Fibromatose, Lupus erythematosus, Hauterkrankungen während Vaskulitis und anderen Angiopathien, Hauttumore, gutartige Hauttumore, Hauterkrankungen der Psoriasis; Hautkomplikationen aufgrund einer Behandlung mit Kortikoiden, Zytostatika, Retinsäure und Derivaten davon oder mit anderen natürlichen Substanzen und/oder synthetischen Substanzen; Verbrennungen I., II., III. und IV. Grades, atopische Dermatitis, Hauterkrankungen der Psoriasis-Arthritis, Phlyktäne, Tylom, Ulcus trophicum, Diabetes-Geschwür, vaskuläre Nekrose der Haut, Analrhagaden, Hämorrhoiden, Geschwüre und/oder Phlogose der Vaginalschleimhaut sind.

18. Zusammensetzung nach einem der Ansprüche 1 bis 10 zur Verwendung als Co-Adjuvans in der Therapie um Hautschäden zu vermeiden, wobei die Therapie ausgewählt ist aus der Gruppe umfassend Therapien, die die Verwendung von Laser oder Ultraviolettstrahlungen vorsehen oder Photochemotherapien, Operationen, Chemotherapie, Strahlentherapie, Behandlungen mit Kortikoiden und/oder anderen natürlichen und/oder synthetischen Substanzen zur topischen Verwendung.

19. Dermatologische/dermal-kosmetische Zubereitung, umfassend die Zusammensetzung nach einem der Ansprüche 1 bis 10 und pharmazeutisch annehmbare Hilfsstoffe.

20. Zusammensetzung, umfassend ein oder mehrere Öle und *Oleum coctus Urticae,* hergestellt nach dem Verfahren der Ansprüche 11 bis 15.

21. Verwendung einer Zusammensetzung nach Ansprüchen 1-10 als eine kosmetische Zusammensetzung zur Behandlung von Hautalterung.

## Revendications

1. Composition comprenant une ou plusieurs huiles et *Oleum coctus Urticae.*

2. Composition selon la revendication 1 comprenant, pour chaque litre d'huile ou de mélange d'huiles, *Oleum coctus Urticae* obtenu à partir de 1 à 500 g de la plante brute, de la partie aérienne, et/ou des racines de l'ortie, ou 10 à 250 g, ou 15 à 185 g, ou 20 à 100 g, ou 25 à 60 g, ou 30 à 50 g, de préférence, pour chaque litre d'huile ou de mélange d'huiles, *Oleum coctus Urticae* obtenu à partir de 35 g de la plante brute, de la partie aérienne, et/ou des racines de l'ortie.

3. Composition selon l'une des revendications 1 ou 2, comprenant également une ou plusieurs cires.

4. Composition selon la revendication 3, comprenant, pour une partie de cire ou de mélange de cires, 7 à 25 parties en poids d'huile ou de mélange d'huiles et *Oleum coctus Urticae* obtenu à partir de 0,10 à 3 parties de la plante brute, de la partie aérienne et/ou des racines de l'ortie, de préférence à partir de 8 à 20 parties d'huile ou de mélange d'huiles et *Oleum coctus Urticae* à partir de 0,30 à 2 parties en poids de l'ortie, de façon encore davantage préférée, à partir de 10 à 15 parties d'huile ou de mélange d'huiles et *Oleum coctus Urticae* à partir de 0,40 à 1 partie de l'ortie.

5. Composition selon l'une des revendications 3 ou 4, comprenant, pour une partie de cire ou de mélange de cires, environ 11,4 parties d'huile ou de mélange d'huiles et *Oleum coctus Urticae* obtenu à partir d'environ 0,44 partie en poids de la plante brute, de la partie aérienne, et/ou des racines de l'ortie.

6. Composition selon l'une des revendications 1 à 5, où lesdites une ou plusieurs huiles sont des huiles végétales choisies dans le groupe comprenant l'huile d'Argan, l'huile d'amande, l'huile de rose musquée, l'huile de karité, l'huile de camomille, l'huile d'avocat, l'huile de citron, l'huile de germe de blé, l'huile de carotte, l'huile de calendula, l'huile de millepertuis, de lierre, de noix de coco, de Macassar, de noisette, de grenade, de lavande coton, d'hélichryse, de baobab, d'abricot, de sésame, de tournesol, de noix, de maïs, d'olive.

7. Composition selon l'une des revendications 1 à 6, où ladite huile est l'huile d'olive.

8. Composition selon l'une des revendications 3 à 7, où lesdites une ou plusieurs cires sont des cires synthétiques ou naturelles, d'origine végétale ou d'origine animale.

9. Composition selon l'une des revendications 3 à 7, où ladite cire est la cire d'abeille.

10. Composition selon l'une des revendications 1 à 9, comprenant également un ou plusieurs autres principes actifs d'origine naturelle ou synthétique.

11. Procédé de préparation d'extraits à partir de plantes brutes, dans lequel la plante est l'ortie, où ledit procédé comprend :
- la fourniture d'une ou de plusieurs huiles et leur chauffage à une température située dans la plage de 140 à 180 °C, de préférence à environ 160 °C ;
- l'ajout de la plante brute, de la partie aérienne, et/ou des racines de l'ortie ;
- la macération d'au moins 1 minute jusqu'à 3 heures, ou jusqu'à 1 heure, ou jusqu'à 40 minutes, ou jusqu'à 20 minutes, ou jusqu'à 10 minutes, de préférence durant environ 3 minutes ;
- la filtration, où le filtrat est ladite composition.

12. Procédé selon la revendication 11, dans lequel ladite plante est l'ortie et ledit extrait est une composition comprenant une ou plusieurs huiles et *Oleum coctus Urticae.*

13. Procédé selon les revendications 11 ou 12, comprenant également, après ladite étape de macération et avant la réalisation de ladite filtration :
- l'ajout d'une ou de plusieurs cires ;
- le mélange de la composition.

14. Procédé selon l'une des revendications 11 à 13, où lesdites une ou plusieurs huiles sont des huiles végétales choisies dans le groupe comprenant l'huile d'Argan, l'huile d'amande, l'huile de rose musquée, l'huile de karité, l'huile de camomille, l'huile d'avocat, l'huile de citron, l'huile de germe de blé, l'huile de carotte, l'huile de calendula, l'huile de millepertuis, de lierre, de noix de coco, de Macassar, de noisette, de grenade, de lavande coton, d'hélichryse, de baobab, d'abricot, de sésame, de tournesol, de noix, de maïs, d'olive.

15. Procédé selon la revendication 13, où lesdites une ou plusieurs cires sont des cires synthétiques ou naturelles, d'origine végétale ou d'origine animale.

16. Composition selon l'une des revendications 1 à 10, pour une utilisation dans le traitement ou la prévention de phénomènes dégénératifs cutanés, ou d'un type pathologique, chez un mammifère.

17. Composition pour une utilisation selon la revendication 16, où lesdits phénomènes sont des infections de la peau et du tissu sous-cutané, des lésions septiques, des lésions dues à une explosion, des piqûres d'insectes, des maladies bulleuses, la dermatite et l'eczéma, des troubles papulo-squameux, l'urticaire et l'érythème, des photodermatoses et des changements cutanés par des radiations ionisantes, des troubles de la pigmentation, l'escarre de décubitus, la fibromatose, le lupus érythémateux, des troubles cutanés durant la vasculite et d'autres angiopathies, des affections malignes de la peau, des tumeurs bénignes de la peau, les troubles cutanés du psoriasis ;
des complications cutanées dues à un traitement avec des corticoïdes, des agents cytostatiques, l'acide rétinoïque et ses dérivés, ou avec d'autres substances naturelles et/ou substances synthétiques ;
les brûlures au degré I, II, III et IV, la dermatite atopique, les troubles cutanés de l'arthrite psoriasique, les phlyctènes, le tylome, l'ulcère trophique, l'ulcère diabétique, la nécrose vasculaire de la peau, les fissures anales, les hémorroïdes, les ulcères et/ou la phlogose des muqueuses vaginales.

18. Composition selon l'une des revendications 1 à 10, pour une utilisation en tant que co-adjuvant dans un traitement de prévention des lésions cutanées, où ledit traitement est choisi dans le groupe comprenant des traitements prévoyant l'utilisation du laser ou de radiations ultraviolettes ou des photochimiothérapies, des chirurgies, une chimiothérapie, une radiothérapie, des traitements avec des corticoïdes et/ou d'autres substances naturelles et/ou synthétiques pour une utilisation topique.

19. Préparation dermatologique/dermato-cosmétique comprenant la composition selon l'une des revendications 1 à 10 et des excipients pharmaceutiquement acceptables.

20. Composition comprenant une ou plusieurs huiles et *Oleum coctus Urticae* préparée selon le procédé des revendications 11 à 15.

21. Utilisation d'une composition selon les revendications 1 à 10 en tant que composition cosmétique pour le traitement du vieillissement de la peau.
